# EUROPEAN PATENT APPLICATION

(11) **EP 4 353 200 A1**
(43) Date of publication of application: **17.04.2024**
(21) Application number: 22851936.9
(22) Date of filing: 22.07.2022
(51) Int. Cl.: A61F 2/82

(54) **STENT FOR BLOOD VESSEL**

(30) Priority: 03.08.2021 CN 202110886550
(71) Applicant: Shanghai MicroPort Medical (Group) Co., Ltd., Shanghai 201203 (CN)
(72) Inventor: WANG, Xueqin, Shanghai 201203 (CN); LI, Jie, Shanghai 201203 (CN); ZHU, Jing, Shanghai 201203 (CN); LIU, Wei, Shanghai 201203 (CN)
(74) Representative: Marks & Clerk LLP
(86) International application number: PCT/CN2022/107497
(87) International publication number: WO 2023/011222

(57) **Abstract**

The present application relates to a stent for a blood vessel. The stent for a blood vessel includes a covering membrane (100) and anchoring members (200) connected to the covering membrane (100). The anchoring members (100) are configured to secure the covering membrane (100) within the blood vessel. The covering membrane (100) is configured to pocket a diseased area of the blood vessel. The anchoring members (200) are configured to sufficiently attach the covering membrane (100) to the intima of the blood vessel when the stent is subjected to blood flow, fixing the covering membrane (100) in the intended implantation position. The covering membrane (100) can pocket the unstable plaque on the blood vessel intima, preventing rupture of the unstable plaque. Additionally, the covering membrane (100) can cause a foreign body reaction inside the body, stimulating endothelial growth in the blood vessel, transforming the unstable plaque into a stable plaque, reducing the incidence of acute thrombosis. Furthermore, this stent can also be used for blood vessel tears occurred in an interventional therapy, preventing the detachment of the torn blood vessel wall and subsequent thrombotic events.

## Description

### CROSS-REFERENCE TO RELATED APPLICATIONS

This application is claims priority to Chinese patent application No. 202110886550.8, filed on August 3, 2021, entitled "STENT FOR BLOOD VESSEL". The contents of the above identified applications are hereby incorporated herein in their entireties by reference.

### TECHNICAL FIELD

This application relates to the field of medical devices, particularly relates to a stent for a blood vessel.

### BACKGROUND

Arteriosclerosis is currently regarded as a chronic inflammatory disease characterized by lipid deposition beneath the intima of arteries, accompanied by proliferation of smooth muscle cells and fibrous matrix components, gradually progressing to form atherosclerotic plaques in specific arterial regions. This process leads to narrowing of the blood vessel lumen or thrombus formation. The plaques gradually enlarge with the chronic progression of the disease, and are categorized as stable or unstable plaques. Unstable plaques are typically a thin and unstable fibrous cap covering a large lipid core, prone to rupture, which can cause thrombus formation and subsequent acute arterial symptoms, posing high risks. In contrast, stable plaques have a thicker fibrous cap, less prone to rupture, clinically presenting symptoms induced by ischemia during physical activities.

Researches indicate that the risks of atherosclerotic thrombus formation and thromboembolic complications seem to be more associated with the stability of atherosclerotic plaques rather than their size. Stable angina is associated with smooth fibrous coronary artery plaques (stable plaques), while unstable angina, acute myocardial infarction (AMI), and sudden cardiac death are almost exclusively linked to plaque instability.

### SUMMARY

In view of the rupture risk of atherosclerotic plaques, there is a need to provide a stent for a blood vessel.

A stent for a blood vessel includes a covering membrane and anchoring members connected to the covering membrane. The anchoring members are disposed at both ends of the stent along an axial direction of the stent and are configured to secure the stent within the blood vessel. The covering membrane is fixed through the anchoring members and is configured to pocket a diseased area of the blood vessel, wherein an outer surface of the covering membrane includes a cell-binding substance.

In an embodiment, the covering membrane is made of a bioabsorbable material, including at least one of a polyester, a polycarbonate, or a composite thereof.

In an embodiment, the porosity of the covering membrane ranges from 50% to 100%.

In an embodiment, the porosity at both ends of the covering membrane, along the axial direction of the stent, is greater than that in the middle section of the covering membrane.

In an embodiment, the thickness of the covering membrane is less than or equal to 100 µm.

In an embodiment, two or more of the anchoring members are located at the same end of the stent and distributed along the axial direction of the stent.

In an embodiment, adjacent two of the anchoring members located at the same end of the stent are spaced apart and connected through a connecting member.

In an embodiment, at least one support member is connected between adjacent two of the anchoring members respectively located at different ends of the stent, and the support member is located on an inner side of the covering membrane.

In an embodiment, the support member is further fixed to the middle section of the covering membrane.

In an embodiment, the length of the support member along the axial direction of the stent is greater than the distance between adjacent two of the anchoring members respectively located at different ends of the stent, thereby arching the covering membrane.

In an embodiment, the length of the support member along the axial direction of the stent is 5% to 20% greater than the distance between adjacent two of the anchoring members respectively located at different ends of the stent.

In an embodiment, the anchoring members located at the ends of the stent are spaced apart from the covering membrane and connected to the covering membrane through mounting members.

In an embodiment, the anchoring members are formed by a straight, wavy, or bent anchoring bar connected head to tail.

In an embodiment, the width of the anchoring member ranges from 50 µm to 5000 µm.

In an embodiment, a region of the anchoring members adjacent to an inner wall of the blood vessel is provided with a groove.

In the above-described blood vessel stent, the anchoring members are configured to sufficiently attach the covering membrane to the intima of the blood vessel when the stent is subjected to blood flow, fixing the covering membrane in the intended implantation position. The covering membrane is configured to pocket the unstable plaque on the blood vessel intima, preventing rupture of the unstable plaque. The outer surface of the covering membrane includes a cell-binding substance. As like dissolves like, a binding force can be created between the covering membrane and the cells of the blood vessel wall, preventing excessive droop of the covering membrane due to insufficient support force. As a result, the diseased area (especially an area with excessive volume or weight) can be adequately pocketed by the covering membrane, effectively preventing rupture of the unstable plaque. Additionally, the covering membrane can cause a foreign body reaction inside the body, stimulating endothelial growth in the blood vessel, transforming the unstable plaque into a stable plaque, reducing the incidence of acute thrombosis. Furthermore, this stent can also be used for blood vessel tears occurred in an interventional therapy, preventing the detachment of the torn blood vessel wall and subsequent thrombotic events.

### BRIEF DESCRIPTION OF THE DRAWINGS

In order to illustrate the embodiments of the present application more clearly, the drawings used in the embodiments will be described briefly. It is evident that the following described figures are merely for some embodiments of the present application, and other figures can be derived by those of ordinary skill in the art without any creative effort.
FIG. 1 is a schematic structural view of a stent for a blood vessel according to an embodiment of the present application.
FIG. 2 is a schematic structural view of a stent for a blood vessel according to another embodiment of the present application.
FIG. 3 is a schematic structural view of a stent for a blood vessel according to another embodiment of the present application.
FIG. 4 is a schematic structural view of a stent for a blood vessel according to another embodiment of the present application.
FIG. 5 is a schematic structural view of a stent for a blood vessel according to another embodiment of the present application.

The reference signs in the figures denote:
100 - covering membrane; 200 - anchoring member; 300 - connecting member; 400 - support member; 500 - mounting member.

### DETAILED DESCRIPTION

To make the objectives, features, and advantages of the present application more understandable, detailed explanations of specific embodiments are provided below, along with accompanying drawings. Many specific details are disclosed in the following description to facilitate a comprehensive understanding of the present application. However, it should be noted that the present application can be implemented in various ways different from those described herein, and those skilled in the art may make similar improvements without departing from the scope of the present application. Therefore, the present application is not limited to the specific embodiments disclosed below.

In the description of the present application, it should be understood that the terms "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial" , "radial", "circumferential", etc. indicate the orientations or positional relationships on the basis of the drawings. These terms are only for describing the present application and simplifying the description, rather than indicating or implying that the related devices or elements must have the specific orientations, or be constructed or operated in the specific orientations, and therefore cannot be understood as limitations of the present application.

In addition, the terms "first" and "second" are merely used for descriptive purposes, and should not be construed as indicating or implying relative importance or implying the quantity of the described technical features. Therefore, the features modified by "first" or "second" may explicitly or implicitly include at least one of the features. In the description of the present application, "a plurality of" means at least two, such as two, three, etc., unless otherwise expressly and specifically defined.

In the present application, unless otherwise clearly specified and defined, the terms "installed", "connected", "coupled", "fixed" and the like should be interpreted broadly. For example, an element, when being referred to as being "installed", "connected", "coupled", "fixed" to another element, unless otherwise specifically defined, may be fixedly connected, detachably connected, or integrated to the other element, may be mechanically connected or electrically connected to the other element, may be directly connected to the other element or connected to the other element via an intermediate element, and may be in internal communication with or in interaction with the other element. For those of ordinary skill in the art, the specific meaning of the above-mentioned terms in the present application can be understood according to specific circumstances.

In the present application, unless otherwise clearly specified and defined, an element, when being referred to as being located "on" or "under" another element, may be in direct contact with the other element or contact the other element via an intermediate element. Moreover, the element, when being referred to as being located "on", "above", "over" another element, may be located right above or obliquely above the other element, or merely located at a horizontal level higher than the other element; the element, when being referred to as being located "under", "below", "beneath" another element, may be located right below or obliquely below the other element, or merely located at a horizontal level lower than the other element.

It should be noted that an element, when being referred to as being "fixed" or "mounted" to another element, may be fixed or mounted to the other element directly or via an intermediate element. An element, when being referred to as being "connected" to another element, may be connected to the other element directly or via an intermediate element. Such terms as "vertical", "horizontal", "up", "down", "left", "right" and the like used herein are for illustrative purposes only and are not meant to be the only ways for implementing the present application.

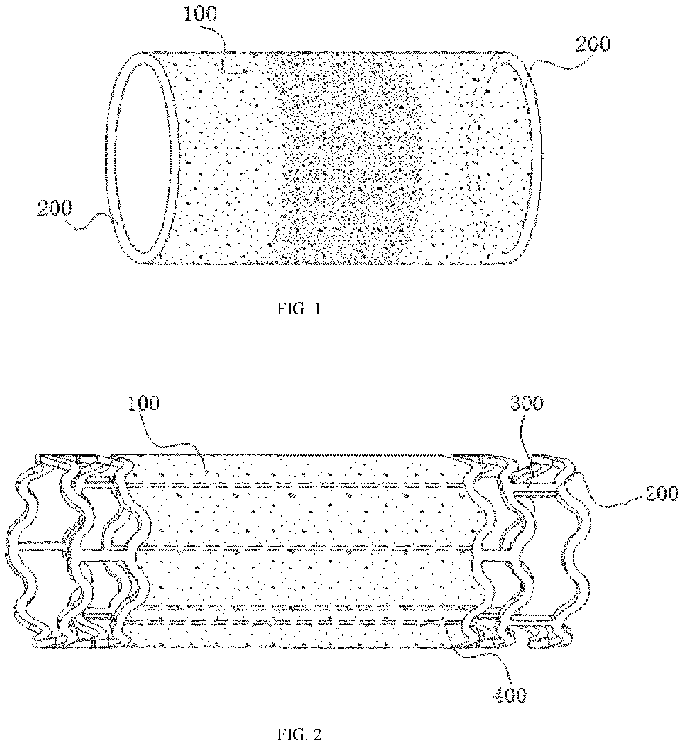

Referring to FIG. 1, FIG. 1 is a schematic structural view of a stent for a blood vessel according to an embodiment of the present application. The embodiment provides a stent for a blood vessel, including a covering membrane 100 and anchoring members 200 connected to the covering membrane 100. The anchoring members 200 are disposed at both ends of the stent along an axial direction of the stent and are configured to secure the stent within the blood vessel. The covering membrane 100 is fixed through the anchoring members 200 and is configured to pocket a diseased area of the blood vessel, wherein an outer surface of the covering membrane 100 includes a cell-binding substance.

In the present embodiment, the diseased area of the blood vessel can be an unstable plaque on the intima of the blood vessel or a diseased area such as a torn area of the intima of the blood vessel that may cause thrombosis. It should be noted that the term "pocket" in this application includes the meaning of hold and capture, so as to provide support to the diseased area on the intima of the blood vessel, such as the unstable plaque, to prevent excessive collapse of the unstable plaque and effectively avoid the rupture of the unstable plaque.

In the present embodiment, the cell-binding substance on the outer surface of the covering membrane 100 is similar to the cellular surface components of the blood vessel wall, thereby generating a binding force with the cells of the blood vessel wall due to the principle that like dissolves like. Consequently, when the diseased area of the blood vessel exceeds normal volume or carries excessive weight, this binding force can prevent excessive droop of the covering membrane 100 due to insufficient support force. As a result, the diseased area can be adequately pocketed by the covering membrane 100. In a preparation process, the cell-binding substance can be directly applied onto the surface of the covering membrane 100 followed by air-drying or desiccation. The cell-binding substance can include one or more of a protein, a physiologically active substance, or a compound.

Examples of the protein include: disease markers such as a carcinoembryonic antigen, a squamous cell carcinoma-related antigen, a cytokeratin 19 fragment, a sialylated sugar chain antigen KL-6, a natriuretic peptide, troponin, myoglobin, etc.; cell growth factors such as interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-18 (IL-18), interleukin-21 (IL-21), interferon-α (IFN-α), interferon-β (IFN-β), interferon-γ (IFN-γ), granulocyte colony-stimulating factor (G-CSF), monocyte colony-stimulating factor (M-CSF), granulocyte-macrophage colony-stimulating factor (GM-CSF), stem cell factor (SCF), flk2/flt3 ligand (FL), leukemia inhibitory factor (LIF), oncostatin M (OM), erythropoietin (EPO), thrombopoietin (TPO), transforming growth factor-α (TGF-α), transforming growth factor-β (TGF-β), macrophage inflammatory protein-1α (MIP-1α), epidermal growth factor (EGF), fibroblast growth factors 1, 2, 3, 4, 5, 6, 7, 8, 9 (FGF-1, 2, 3, 4, 5, 6, 7, 8, 9), neuronal growth factor (NGF), hepatocyte growth factor (HGF), leukemia inhibitory factor (LIF), protease nexin I, protease nexin II, platelet-derived growth factor (PDGF), cholinergic differentiation factor (CDF), chemokines, Notch ligands (Delta 1, etc.), Wnt proteins, angiopoietin-like proteins 2, 3, 5, 7 (Angpt 2, 3, 5, 7), insulin-like growth factor (IGF), insulin-like growth factor binding protein (IGFBP), pleiotrophin, insulin, growth hormone, etc.; cell adhesion factors such as collagen types I to XIX, fibronectin, vitronectin, laminins 1 to 12, laminin 511, laminin 521, nitogen (Japanese: 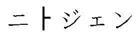 ), tenascins, thrombospondins, Von Willebrand factor, osteopontin, fibrinogen, various elastin, various proteoglycans, various cadherins, desmocolins, desmogleins, various integrins, E-selectin, P-selectin, L-selectin, immunoglobulin superfamily, matrigel, poly-D-lysine, poly-L-lysine, etc.; as well as various antibodies such as IgG, IgM, IgA, IgD, IgE, etc.

Examples of the physiologically active substance include: carbohydrates such as D-glucosamine, D-galactosamine, neuraminic acid, hyaluronic acid, chondroitin sulfate, heparan sulfate, heparin, etc.; serotonin, norepinephrine, epinephrine, 3-(3,4-dichlorophenyl)-1,1-dimethylurea (DCMU), atrazine, linuron, simazine, etc.

Examples of the compound include: peptides such as angiotensins I to IV, bradykinin, fibrinopeptides, natriuretic peptides, urodilatin, guanylin peptide, endothelins 1 to 3, salusin, urotensins, oxytocin, neurophysin, vasopressin, adrenocorticotropic hormone, melanocyte-stimulating hormone, endorphins, lipotropin, urocortins 1 to 3, luteinizing hormone-releasing hormone, growth hormone-releasing hormone, somatostatin, cortistatin, prolactin-releasing peptide, metastin, tachykinin, substance P, neurokinin, endokinin, neurotensin, neuromedin, peptide from *Eisenia bicyclis* (Japanese: 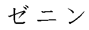 ), ghrelin, obestatin, melanin-concentrating hormone, orexin, neuropeptide, dynorphin, neoendorphin, endomorphin, nociceptin, pyroglutamylated RF-amide peptide, galanin, gastrin, cholecystokinin, secretin, relaxin, glucagon, glicentin, adrenomedullin, amyrin, calcitonin, parathyroid hormone, defensin, thymosin, YIGSR peptide, etc.; amino acids such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, cystine, hydroxyproline, hydroxylysine, dihydroxyphenylalanine, thyroxine, phosphoserine, desmosine, β-alanine, sarcosine, omithine, creatine, γ-aminobutyric acid, theanine, kainic acid, domoic acid, ibotenic acid, etc.; primary amines such as 2-(dimethylamino)ethylamine (CAS No.: 108-00-9), N-(2-hydroxyethyl)ethylenediamine (CAS No.: 111-41-1), N-(2-aminoethyl)piperazine (CAS No.: 140-31-8), 4-(2-aminoethyl)morpholine (CAS No.: 2038-03-1), 1-(2-aminoethyl)-2-imidazolidinone (CAS No.: 6281-42-1), tryptamine (CAS No.: 61-54-1), histamine dihydrochloride (CAS No.: 56-92-8), tyramine (CAS No.: 51-67-2), dopamine (CAS No.: 51-61-6), etc.; primary diamines such as ethylenediamine dihydrochloride (CAS No.: 333-18-6), 1,6-diaminohexane (CAS No.: 124-09-4), N,N'-bis(aminopropyl)piperazine (CAS No.: 7209-38-3), etc.

In the above-described blood vessel stent, the anchoring members 200 are configured to sufficiently attach the covering membrane 100 to the intima of the blood vessel when the stent is subjected to blood flow, fixing the covering membrane 100 in the intended implantation position. The covering membrane 100 is configured to pocket the unstable plaque on the blood vessel intima. The outer surface of the covering membrane 100 includes a cell-binding substance. As like dissolves like, a binding force can be created between the covering membrane 100 and the cells of the blood vessel wall, preventing excessive droop of the covering membrane 100 due to insufficient support force. As a result, the diseased area (especially an area with excessive volume or weight) can be adequately pocketed by the covering membrane 100, effectively preventing rupture of the unstable plaque. Additionally, the covering membrane 100 can cause a foreign body reaction inside the body, stimulating endothelial growth in the blood vessel, transforming the unstable plaque into a stable plaque, reducing the incidence of acute thrombosis. Furthermore, this stent can also be used for blood vessel tears occurred in an interventional therapy, preventing the detachment of the torn blood vessel wall and subsequent thrombotic events.

In patients with carotid atherosclerotic diseases, the irregularity and the rupture of the plaques are closely associated with cerebral ischemic events. Patients with irregular or ulcerative plaques exhibit a higher risk of ischemic stroke, regardless of the degree of luminal narrowing. In view of this issue, in some embodiments of the present application, the material of the covering membrane 100 is a flexible material. Such a material allows the covering membrane 100 to pocket various shapes of unstable plaques, especially irregularly shaped unstable plaques, thereby reducing the high risk of ischemic stroke in patients with irregularly shaped plaques. Optionally, the covering membrane 100 is made of a bioabsorbable material, including at least one of a polyester (such as polycaprolactone, polylactic acid, polyethylene glycol), a polycarbonate, or a composite thereof. The bioabsorbable material of the covering membrane 100 can degrade within and be absorbed by the human body.

In some embodiments of the present application, the porosity of the covering membrane 100 ranges from 50% to 100%, for example, is 50%, 55%, 60%, 65%, 70%, 75%, 80%, 85%, 90, 95%, 100%, etc. In this way, it can be ensured that the covering membrane 100 can pocket the diseased area and the covering membrane 100 is flexible.

Specifically, the porosity at both ends of the covering membrane 100, along the axial direction of the stent, is greater than that in the middle section of the covering membrane 100. The middle section of the covering membrane 100 refers to the portion located between the ends of the covering membrane 100 along the axial direction of the stent. It can be understood that the covering membrane 100 includes two ends (i.e., the first end and the second end) and the middle section. The first end, the middle section, and the second end are sequentially distributed along the axial direction of the stent, following the direction of blood flow. The middle section of the covering membrane 100 is configured to pocket the diseased area of the blood vessel, requiring higher density, while the ends of the covering membrane 100 are configured for mounting the anchoring member 200, having lower density requirements, and can have a lower density which can save materials and reduce processing difficulty. During application, the porosities at the ends and the middle section of the covering membrane 100 can be adjusted according to specific requirements.

In some embodiments of the present application, the thickness of the covering membrane 100 is less than or equal to 100 µm, for example, can be 100 µm, 90 µm, 80 µm, 70 µm, 60 µm, etc. This facilitates the cells extending into the middle section of the covering membrane 100, expediting endothelialization of the blood vessel, offers increased flexibility, and occupies less space within the blood vessel lumen. Preferably, the thickness of the covering membrane 100 is less than or equal to 50 µm, for example can be 50 µm, 40 µm, 30 µm, 20 µm, 10 µm, etc.

In some embodiments of the present application, the covering membrane 100 can be processed and formed through techniques such as weaving, electrospinning, lamination, or hollow cutting. During application, an appropriate processing method for forming the covering membrane 100 can be selected based on the material of the covering membrane 100.

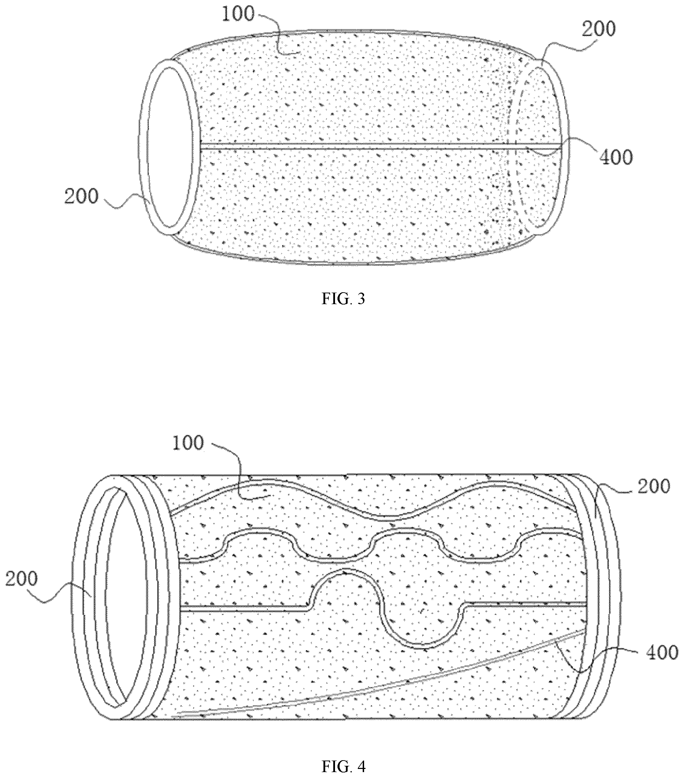

In some embodiments of the present application, as shown in FIG. 1, multiple anchoring members 200 are located at the same end of the stent and are sequentially distributed along the axial direction of the stent. It can be understood that the stent includes two ends (i.e., the first end and the second end), which are sequentially distributed along the axial direction of the stent, following the direction of blood flow. The term "same end of the stent" refers to either the first end or the second end of the stent. Having multiple anchoring members 200 disposed at the same end of the stent can enhance the stability of implantation of the covering membrane 100 within the blood vessel. During application, the specific number of the anchoring members 200 disposed on the same end of the stent can be determined based on requirements. For example, as shown in FIG. 2, two anchoring members 200 are disposed at one end of the covering membrane 100, while three anchoring members 200 are disposed at the other end of the covering membrane 100. As another example shown in FIG. 3, three anchoring members 200 are disposed at each end of the covering membrane 100. In other embodiments of the present application, the length of the anchoring member 200 along the axial direction of the stent can be increased to enhance the anchoring strength of the anchoring member 200. In such cases, as shown in FIG. 4, adjacent anchoring members 200 at the same end of the covering membrane 100 are in contact with and connected to each other. The adjacent anchoring members 200 can be connected by using fusion welding, integrated molding (e.g., laser engraving), or other methods.

In further embodiments of the present application, as shown in FIG. 2, adjacent two anchoring members 200 located at the same end of the covering membrane 100 are spaced apart and connected by a connecting member 300. The term "same end of the covering membrane 100" refers to either the first end or the second end of the covering membrane 100. This arrangement allows the anchoring members 200 to form a skeleton structure, accelerating the endothelialization process of the blood vessel. Moreover, a blood vessel transport system can more effortlessly grip the anchoring members 200, facilitating the delivery of the stent. Optionally, the material of the connecting member 300 can be the same as that of the anchoring member 200. The connecting member 300 can be of various shapes such as straight, bent, or wavy. The connecting member 300 can be connected to the anchoring member 200 using fusion welding, integrated molding (e.g., laser engraving), or other methods.

In some other embodiments of the present application, regions of the anchoring members 200 adjacent to the inner wall of the blood vessel are provided with grooves. Cells can be grown in the grooves, thereby firmly fixing the anchoring members 200 within the blood vessel and further enhancing the stability of the implantation of the covering membrane 100 within the blood vessel. The shape of the grooves can be circular, polygonal, etc., which are not limited in the embodiments of the present application. It is to be noted that when the anchoring members 200 are ring-shaped, the surface of the anchoring members 200 facing the inner wall of the blood vessel is the region of the anchoring members 200 adjacent to the inner wall of the blood vessel, and the surface of the anchoring members 200 facing away from the inner wall of the blood vessel is the region of the anchoring members 200 adjacent to the central axis of the stent. When the anchoring member 200 includes multiple protrusions, such as wedge-shaped protrusions, arranged around the central axis of the stent, and the protrusions are inserted into the inner wall of the blood vessel for anchoring, the protrusion surface that is inserted into the inner wall of the blood vessel is the region of the anchoring members 200 away from the central axis of the stent, and the protrusion surface that is not inserted into the inner wall of the blood vessel is the region of the anchoring members 200 adjacent to the inner wall of the blood vessel. The central axis of the stent extends parallel to the direction of blood flow.

In some embodiments of the present application, the anchoring members 200 are ring-shaped structures. The completely closed anchoring members 200 ensure a well-fitted attachment of the covering membranes 100 to the inner wall of the blood vessel.

Specifically, the anchoring member 200 is formed by connecting the head to the tail of an anchoring bar in a straight (referring to FIGs. 1 and 3 to 5), wavy (referring to FIG. 2), or bent shape. The anchoring member 200 formed from the straight anchoring bar connected head to tail can reduce the manufacturing complexity of the stent for the blood vessel. The anchoring member 200 formed from the wavy or bent anchoring bar connected head to tail can facilitate compression of the stent as a whole for easier implantation. In application, the anchoring members 200 can be configured in various shapes for effective pocketing different sizes of diseased areas.

In some embodiments of the present application, the width of the anchoring member 200 ranges from 50 µm to 5000 mm, for example, can be 50 µm, 100 µm, 1000 µm, 3000 µm, or 5000 µm. The width of the anchoring member 200 is greater that of a metal strut in a traditional coated stent, allowing the anchoring member 200 in this size to effectively secure the covering membrane 100 within the blood vessel. It is to be noted that the greater the number of anchoring members 200 on the covering membrane 100, the narrower the width of each anchoring member 200 correspondingly.

In some embodiments of the present application, the material of the anchoring members 200 includes at least one of nickel-titanium alloy, cobalt-chromium alloy, stainless steel, zinc-iron alloy, magnesium alloy, polyester, polycarbonate, or a composite thereof. Specifically, when the material of the anchoring members 200 is nickel-titanium alloy or shape-memory polyester (such as polyurethane), the anchoring members 200 can self-expand. This eliminates the need for tools (e.g., balloons) to perform balloon expansion of the anchoring members 200, reducing surgical complexity and minimizing the risk of damaging the diseased area of the blood vessel. In cases where other materials are used for the anchoring members 200, the anchoring members 200 may not self-expand and require tools (e.g., balloons) for balloon expansion of the anchoring members 200. Additionally, in the cases where balloon expansion is performed, the material of the anchoring members 200 may also possess shape-memory ability, enabling outward tension of the anchoring members 200 to achieve an enhanced anchoring effect.

In some embodiments of the present application, as shown in FIGs. 2 to 5, at least one support member 400 is disposed between adjacent anchoring members 200 respectively located at different ends of the covering membrane 100. The support member 400 is located on an inner side of the covering membrane 100. During the expansion of the covering membrane 100 by the anchoring member 200, the support member 400 can also assist to expand the covering membrane 100. The support member 400 not only reduces the radial collapse of the covering membrane 100 but also reduces the axial retraction of the stent.

Optionally, the material of the support member 400 can be the same as or different from the material of the anchoring members 200. The support member 400 can extend along the axial direction of the stent for the blood vessel, or can extend at an angle (e.g., 5°, 10°, 20°, etc.) relative to the axial direction of the stent. The shape of the support member 400 can be straight, bent, wavy, or helical. The support member 400 can be connected to the corresponding anchoring members 200 using fusion welding, integrated molding (e.g., laser cutting), or other methods.

Optionally, the support member 400 can also be fixed to the middle section of the covering membrane 100, so that the support member 400 can effectively assist in expanding the covering membrane 100 while the anchoring member 200 expanding the covering membrane 100. The support member 400 can be fixed to the middle section of the covering membrane 100 using weaving, bonding, fusion welding, or other methods.

Optionally, as shown in FIG. 4, the length of the support member 400 along the axial direction of the stent is greater than the distance between adjacent anchoring members 200 respectively located at different ends of the covering membrane 100, so as to support the covering membrane 100, preventing the covering membrane 100 from drooping due to excessive length and allowing the covering membrane 100 to sufficiently pocket a relatively large unstable plaque. Optionally, the length of the support member 400 along the axial direction of the stent is greater than the distance between adjacent anchoring members 200 respectively located at different ends of the stent by 5% to 20%, such as 5%, 10%, 15%, 20%, etc., avoiding the covering membrane 100 being too tight when it is arched due to the support member 400 being too short, while also preventing any gaps between the stent and the inner wall of the blood vessel due to the excessive length of the support member 400.

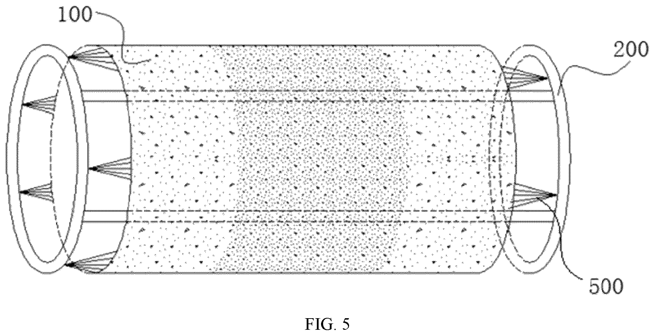

In some embodiments of the present application, as shown in FIG. 5, the anchoring members 200 adjacent to the ends of the covering membrane 100 are spaced apart from the covering membrane 100 and connected to the covering membrane 100 through mounting members 500. This configuration reduces the coverage of the covering membrane 100, allowing cells to grow to the central area of the covering membrane 100, promoting endothelialization. Optionally, the mounting members 500 can be threads, such as filaments. Along the circumferential direction, a plurality of filaments can be pulled out from the ends of the covering membrane 100. Alternatively, in other embodiments of the present application, the anchoring members 200 adjacent to the ends of the covering membrane 100 can be fixed directly onto the covering membrane 100 by using weaving, fusion welding, or other methods. In application, the method for connecting the covering membrane 100 to the anchoring members 200 adjacent to the ends of the covering membrane 100 can be selected based on the location of the diseased area and the size of the covering membrane 100.

### Example 1

This example provides a stent for a blood vessel. As shown in FIG. 2, the stent includes two anchoring members 200 at one end of the stent and three anchoring members 200 at the other end of the stent. The anchoring members 200 at the same end are connected by straight connecting members 300. The material of the anchoring members 200 is cobalt-chromium alloy, and the anchoring members 200 are laser-cut into a wavy shape. The connecting members 300 are also formed on the anchoring members 200 using laser cutting. A covering membrane 100 made of polycaprolactone (PCL) is connected between two adjacent anchoring members 200 respectively located at different ends of the stent by weaving and knotting. The covering membrane 100 has a thickness of 20 µm and is formed by electrospinning. Additionally, four poly-L-lactic acid (PLLA) strands with a diameter of 10 µm as the support members 400 are disposed at equal intervals between the two adjacent anchoring members 200 respectively located at different ends of the stent. The PLLA strands are located on the inner side of the covering membrane 100, assisting in supporting the covering membrane 100 during expansion of the stent.

### Example 2

This example provides a stent for a blood vessel. As shown in FIG. 3, the blood vessel stent includes one injection-molded polycarbonate anchoring member 200 at each end. A support member 400 made of nickel-titanium alloy, with a width of 50 µm and a thickness of 10 µm, is connected between the two anchoring members 200. The support member 400 winds around the anchoring members 200 in a single loop and then fusion welded onto the anchoring members 200. The support member 400 is in a straight shape and extends along the axial direction of the stent. The length of the support member 400 is 5% to 20% greater than the distance between the anchoring members 200. When the stent is released, the support member 400 slightly protrudes outward, tightening the covering membrane 100 to prevent collapse.

### Example 3

This example provides a stent for a blood vessel. As shown in FIG. 4, the stent includes three anchoring members 200 at each end of the stent, and adjacent anchoring members 200 at the same end are attached together. Four bent support members 400 are disposed between two adjacent anchoring members 200 respectively located at different ends of the stent. The support members 400 and the anchoring members 200 are formed by laser-engraving a polylactic acid tube. A covering membrane 100 made of polylactic acid is connected between the two adjacent anchoring members 200 respectively located at different ends of the stent by weaving. The molecular weight of the polylactic acid of the anchoring members 200 is above 200,000, higher than the molecular weight of the polylactic acid of the covering membrane 100, providing sufficient support. Additionally, the anchoring members 200 are engraved with grooves to increase the fixation strength.

### Example 4

This example provides a stent for a blood vessel. As shown in FIG. 5, the stent includes one anchoring member 200 at each end of the stent. Straight support members 400 are disposed between the two anchoring members 200, increasing the support for the covering membrane 100. Both the support members 400 and anchoring members 200 are made of cobalt-chromium alloy. A covering membrane 100 made of a mixture of PCL and PGLA is connected between the two anchoring members 200 by weaving. The covering membrane 100 has a lower porosity in the middle section and a higher porosity at the ends. Five braided filaments as the mounting members 500 are drawn out from each end of the covering membrane 100, which are tethered to the anchoring member 200.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A stent for a blood vessel, comprising a covering membrane (100) and anchoring members (200) connected to the covering membrane; wherein
the anchoring members (200) are disposed at both ends of the stent along an axial direction of the stent and are configured to secure the stent within the blood vessel;
the covering membrane (100) is fixed through the anchoring members (200) and is configured to pocket a diseased area of the blood vessel, wherein an outer surface of the covering membrane (100) comprises a cell-binding substance.

2. The stent according to claim 1, wherein the covering membrane (100) is made of a bioabsorbable material selected from the group consisting of a polyester, a polycarbonate, and a composite thereof.

3. The stent according to claim 1, wherein a porosity of the covering membrane (100) ranges from 50% to 100%.

4. The stent according to any one of claims 1 to 3, wherein the porosity at both ends of the covering membrane (100), along the axial direction of the stent, is greater than that in a middle section of the covering membrane (100).

5. The stent according to any one of claims 1 to 3, wherein a thickness of the covering membrane (100) is less than or equal to 100 µm.

6. The stent according to any one of claims 1 to 3, wherein two or more of the anchoring members (200) are located at a same end of the stent and distributed along the axial direction of the stent.

7. The stent according to claim 6, wherein adjacent two of the anchoring members (200) located at the same end of the stent are spaced apart and connected through a connecting member (300).

8. The stent according to any one of claims 1 to 3, further comprising at least one support member (400) connected between adjacent two of the anchoring members (200) respectively located at different ends of the stent, and the support member (400) is located on an inner side of the covering membrane (100).

9. The stent according to claim 8, wherein the support member (400) is further fixed to a middle section of the covering membrane (100).

10. The stent according to claim 8, wherein a length of the support member (400) along the axial direction of the stent is greater than a distance between the adjacent two of the anchoring members (200) respectively located at different ends of the stent, thereby arching the covering membrane (100).

11. The stent according to claim 10, wherein the length of the support member (400) along the axial direction of the stent is 5% to 20% greater than the distance between the adjacent two of the anchoring members (200) respectively located at different ends of the stent.

12. The stent according to any one of claims 1 to 3, wherein the anchoring members (200) located at the ends of the stent are spaced apart from the covering membrane (100) and connected to the covering membrane (100) through mounting members (500).

13. The stent according to any one of claims 1 to 3, wherein the anchoring members (200) are formed by a straight, wavy, or bent anchoring bar connected head to tail.

14. The stent according to any one of claims 1 to 3, wherein widths of the anchoring members (200) range from 50 µm to 5000 µm.

15. The stent according to any one of claims 1 to 3, wherein a region of the anchoring members (200) adjacent to an inner wall of the blood vessel is provided with a groove.
